# EUROPÄISCHE PATENTSCHRIFT

(11) **EP 2 115 453 B1**
(45) Veröffentlichungstag und Bekanntmachung des Hinweises auf die Patenterteilung: **12.04.2017**
(21) Anmeldenummer: 08708381.2
(22) Anmeldetag: 29.01.2008
(51) Int. Cl.: G01N 31/12, G01N 33/18

(54) **VERFAHREN UND VORRICHTUNG ZUR BESTIMMUNG DES PHOSPHORGEHALTES EINER WÄSSRIGEN PROBE**
METHOD AND DEVICE FOR DETERMINING THE PHOSPHORUS CONTENT OF AN AQUEOUS SAMPLE
PROCÉDÉ ET DISPOSITIF POUR DÉTERMINER LA TENEUR EN PHOSPHORE D'UN ÉCHANTILLON AQUEUX

(30) Priorität: 29.01.2007 DE 102007004339
(43) Veröffentlichungstag der Anmeldung: 11.11.2009
(73) Patentinhaber: LAR Process Analysers AG, 10179 Berlin (DE); Arts, Werner, 19825 Berlin (DE)
(72) Erfinder: GENTHE, Wolfgang, 14059 Berlin (DE)
(74) Vertreter: Heinze, Ekkehard
(86) Internationale Anmeldenummer: PCT/EP2008/051064
(87) Internationale Veröffentlichungsnummer: WO 2008/092869

(56) Entgegenhaltungen:
- DE-A1- 2 728 706
- DE-B3- 10 240 410
- COLOMBINI S ET AL: "Use of column-switching ion chromatography for the simultaneous determination of total nitrogen and phosphorus after microwave assisted persulphate digestion" JOURNAL OF CHROMATOGRAPHY, ELSEVIER SCIENCE PUBLISHERS B.V. AMSTERDAM, NL, Bd. 822, Nr. 1, 25. September 1998 (1998-09-25), Seiten 162-166, XP004140652 ISSN: 0021-9673
- LAMBERT D ET AL: "An evaluation of the efficiency of the alkaline persulphate digestion method for the determination of total phosphorus in turbid waters" WATER RESEARCH, ELSEVIER, AMSTERDAM, NL, Bd. 29, Nr. 1, 1. Januar 1995 (1995-01-01), Seiten 7-9, XP004035490 ISSN: 0043-1354
- A. GASPARATOS ET. AL.: "A comparison of wet oxidation methods for determination of total phosphorus in soils" JOURNAL OF PLANT NUTRITION AND SOIL SCIENCE, Bd. 164, 2001, Seiten 435-439, XP002478861

## Beschreibung

Die Erfindung betrifft ein Verfahren zur Bestimmung des Phosphorgehaltes einer wässrigen Probe gemäß dem Oberbegriff des Anspruchs 1 sowie eine Vorrichtung zur Durchführung des Verfahrens.

Es ist bekannt, zur Bestimmung des Gehaltes an bestimmten Wasserinhaltsstoffen - und damit der Qualität von Wasser und insbesondere von durch organische Stoffe, Stickstoffverbindungen und/oder Halogenverbindungen belastetem Abwasser-eine Probe in einer Atmosphäre eines mit Sauerstoff angereicherten inerten Transportgases (Trägergases) zu verdampfen und zu verbrennen und das hierbei erhaltene Verbrennungsgasgemisch einem zum Nachweis von Kohlendioxid, Stickoxiden etc. geeigneten Detektor zuzuführen.

Als Detektoren haben sich (neben anderen) Infrarotdetektoren für den Kohlenstoffgehalt, spezielle Chemolumineszenzdetektoren respektive elektrochemische Sensoren für den Stickstoffgehalt und sogenannte coulometrische Detektoren für den Halogenidgehalt bewährt.

Große Verbreitung haben die auf der Verbrennung einer Wasserprobe beruhenden Nachweisverfahren für die Erfassung des Gehaltes an organischen Inhaltsstoffen - des sogenannten TOC (total organic carbon) - erlangt. Hierbei wird üblicherweise eine kleine Wassermenge mit dem Transportgas einem mit einer Widerstandsheizung auf eine vorbestimmte Temperatur aufgeheizten Ofen zugeführt, wo sie nahezu schlagartig verdampft und verbrennt, und das Verbrennungsgas wird einem NDIR-CO₂-Detektor zugeführt, dessen CO₂-Gehaltsanzeige ein Maß für den C-Gehalt des Wasserprobe bildet. Eine fortgeschrittene Ausführung dieses Verfahrens und eine entsprechende Apparatur sind in DE 43 44 441 C2 beschrieben. Eine zur Messung sehr niedriger TOC-Werte - etwa in hochreinem Wasser bzw. hochreinen Lösungen für medizinische Anwendungen - modifizierte Anordnung ist in Weiterentwickelte Verfahren dieser Art und zweckmäßig ausgestaltete Reaktoren bzw. Gesamtanordnungen hat die Anmelderin in der EP 0 887 643 B1 und der EP 1 055 927 B1 vorgeschlagen.

Neben den oben erwähnten Wasserinhaltstoffen ist auch Phosphor ein chemisches Element, dessen Anteil die Qualität und die anzuwendenden Aufbereitungstechniken von Abwässern wesentlich beeinflussen kann und dessen quantitativer Erfassung daher seit einiger Zeit verstärktes Augenmerk gewidmet wird. Anders als die Wasserinhaltstoffe Kohlenstoff, Stickstoff und Halogenide, lässt sich ein quantitativer Nachweis von Phosphor aber bislang nicht in einem gasförmigen Medium (Verbrennungsgas + Trägergas) vornehmen, sondern hierfür haben sich Nachweismethoden bewährt, die an einer wässrigen Lösung ausgeführt werden. Diese Verfahren nützen, vereinfacht gesagt, den Farbumschlag einer mit einem speziellen Reagens versetzten wässrigen Probe und sind als "Blau-Methode" bzw. "Geib-Methode" bekannt und in entsprechenden Standards fixiert; vgl. hierzu etwa www.wtw.com/media.

Während der Phosphor in natürlichem Wasser in drei Fraktionen auftritt, nämlich als (1) anorganisches, gelöstes Orthophosphat, (2) gelöste organische Phosphorverbindungen und (3) in Biomasse gebundener oder an Partikeln anlagernder partikulärer Phosphor, basieren die bekannten Nachweisverfahren auf der Messung des Orthophosphat-Gehaltes. Zur Bestimmung des Gesamt-Phosphorgehalts einer wässrigen Probe ist daher ein Aufschluss der Fraktionen (2) und (3), dass heißt deren Überführung in photometrisch bestimmbares Orthophosphat, erforderlich.

Dieser Aufschluss erfolgt gemäß einer seit langem bekannten Methode durch Oxidation unter Zugabe von Chemikalien im sauren Medium, ggfs. mit Überdruck und bei erhöhter Reaktionstemperatur, vgl. hierzu etwa JP 2004093509 A. Zur Bestimmung des Phosphorgehaltes in einem organischen Material (etwa Öl) ist auch deren Vermischung mit einer alkalischen Lösung und die Verbrennung unter Sauerstoffatmosphäre in einem hermetisch geschlossenen Reaktionsgefäß bekannt; vgl. JP 62003643 A.

Die US 5702954 beschreibt ein mehrstufiges Aufschlussverfahren für pflanzliche oder tierische phosphorhaltige Proben, welches eine Verbrennung in Gegenwart eins Reduktionsmittels (etwa Wasserstoff) und eine anschließende Umsetzung mit Ozon in einer weiteren Reaktionskammer bei Umgebungstemperatur einschließt. Auch die US 2003/0032194 A1 beschreibt ein mehrstufiges Oxidationsverfahren, welches primär zur Bestimmung von Stickstoff und Schwefel, aber auch von Phosphor, in einer diese Elemente enthaltenden Probe entwickelt wurde. Thermische Aufschlussverfahren unter Einsatz spezieller Katalysatoren bzw. von Ozon sind etwa auch aus der JP 59154358 A oder JP 61140863 A bekannt.

Bekannt sind auch verschiedene Aufschlussverfahren, die sich einer photooxidativen Zersetzung der Probe unter UV-Bestrahlung, insbesondere in Gegenwart eines Photooxidations-Katalysators, bedienen. Verfahren dieser Art sind etwa beschrieben in der EP 0 634 646 B1 oder JP 07027706 A.

Das Dokument DE10240410 beschreibt ein Verfahren zum Aufschluß einer wässrigen Probe, wobei der Aufschluß einstufig durch katalysator-freie Verbrennung als Batch-Aufschluß in einem Verbrennungsofen ausgeführt wird. Das Verfahren dient zur Bestimmung des Sauerstoffbedarfs für einen Klärprozess.

Der Erfindung liegt die Aufgabe zu Grunde, ein verbessertes Verfahren und eine verbesserte Vorrichtung zur Bestimmung von Phosphor in einer wässrigen Probe bereitzustellen, welche einen verfahrenstechnisch unaufwändigen und im Hinblick auf den Einsatz von Verbrauchsmaterialien sparsamen und somit kostengünstigen Aufschluss der Proben erlauben und in der Praxis leicht handhabbar sind.

Diese Aufgabe wird in ihrem Verfahrensaspekt durch ein Verfahren mit den Merkmalen des Anspruchs 1 und in ihrem Vorrichtungsaspekt durch ein Verfahren mit den Merkmalen des Anspruchs 11 gelöst. Zweckmäßige Fortbildungen des Erfindungsgedankens sind Gegenstand der abhängigen Ansprüche.

Die Erfindung schließt den wesentlichen überraschenden Gedanken ein, den Probenaufschluss, dass heißt die Umwandlung der verschiedenen Phosphor-Fraktionen in photometrisch nachweisbares Orthophosphat, einstufig und ohne Katalysator durch Verbrennung einer Probe in einem Verbrennungsofen (also in Gegenwart von Sauerstoff) ausführen zu können. Diese Verbrennung erfolgt als Batch-Aufschluss, dass heißt durch Zuführen einer vorbestimmten kleinen Probenmenge in den (ansonsten hermetisch abgeschlossenen) Ofen in einem Einspritzvorgang.

Des Weiteren schließt die Erfindung den Gedanken ein, das entstehende Verbrennungsgas in einem Trägergasstrom aus dem Verbrennungsofen abzuführen und den Gasstrom zur Gewinnung der benötigten wässrigen Analyse-Lösung derart zu kühlen, dass sich daraus ein praktisch den gesamten Phosphorgehalt der Probe in Form von Orthophosphat enthaltendes Kondensat abscheidet.

Die Erfindung bietet die wesentlichen Vorteile eines vom Ablauf her einfachen und leicht steuerbaren Verfahrens, das mit einer geringen Anzahl und Menge von Verbrauchsmaterialen (chemischen Einsatzstoffen) auskommt und unter Arbeits- und Umweltschutzaspekten als vorteilhaft einzuschätzen ist.

In einer vorteilhaften Ausgestaltung des Verfahrens wird die aufgeschlossene Probe nicht nur zur Phosphor-Bestimmung, sondern auch zur Bestimmung weiterer Wasserinhaltstoffe benutzt, insbesondere zur Kohlenstoff- und/oder Stickstoffbestimmung. Sie wird hierfür einem jeweils geeigneten Detektor zugeführt, also zur Kohlenstoffbestimmung einem CO₂-Detektor üblicher Bauart (NDIR-Detektor) und zur Stickstoffbestimmung einem herkömmlichen NO-Detektor (Chemolumineszenz-Detektor oder elektrochemischer Sensor). Hierfür kann der gleiche Verbrennungs-Trägergasstrom genutzt werden, aus dem das Kondensat zur Bereitstellung der wässrigen Analyse-Lösung abgezogen wurde; in einer alternativen Verfahrensführung können aber auch verschiedene Proben und jeweilige Aufschlussvorgänge einerseits für die Phosphor-Bestimmung und andererseits für die C/N-Bestimmung vorgesehen sein. Eine spezielle Verfahrensführung kann eine alternierende P- und C/N-Bestimmung an aufeinanderfolgend thermisch aufgeschlossenen Messproben umfassen.

In einer weiteren Ausführung der Erfindung ist vorgesehen, dass das Kondensat in einer Kühlfalle gesammelt und aus dieser eine definierte Menge für die photometrische Bestimmung abgezogen wird. Eine besonders variable und zur Handhabung kleiner Probenmengen gut geeignete Ausgestaltung sieht vor, dass das Abziehen von Kondensat aus der Kühlfalle und der Transport an einen Detektionsort mittels einer, insbesondere koordinatengesteuerten und/oder motorisch betätigten, Injektionsspritze ausgeführt wird.

Der Einsatz einer Injektionsspritze hat im Übrigen den Vorteil, dass mit dieser auch eine Vermischung des abgezogenen Kondensats mit einem für den photometrischen Nachweis beizumischenden chemischen Reagens geschehen kann, indem einfach das Reagens nach dem Kondensat in die Spritze gezogen und erforderlichenfalls durch mehrfache Betätigung des Kolbens mit dem Kondensat vermischt wird. Zweckmäßigerweise wird die wässrige Analyse-Lösung dann einer Durchflussküvette zur Ausführung eines der an sich bekannten Analyseverfahren zugeführt. Alternativ hierzu kann das Kondensat auch in einen Reagens-Strom eingespritzt werden.

Der wesentliche Schritt des Kühlens des Verbrennungsgas-/Trägergasstromes kann in genau und einfach auf elektrischem Wege steuerbarer Weise mittels eines Peltier-Kühlers ausgeführt werden. Des Weiteren kann vorgesehen sein, dass der Verbrennungsgas-/Trägergasstrom zweistufig abgekühlt wird, wobei in einer ersten Stufe das Kondensat gewonnen wird und in einer zweiten Stufe eine Abkühlung bis nahe 0°C (etwa auf 2-4°C) erfolgt.

Vorrichtungsseitig gehört zur Erfindung der wesentliche Gedanke, stromabwärts eines an sich herkömmlich ausgebildeten Verbrennungsofens einen Gaskühler mit Kühlfalle zur Abschaltung von Kondensat aus dem Verbrennungsgas-Trägergasstrom sowie Mittel zum Abziehen von Kondensat aus dieser und zu dessen Transport zu einem P-Detektionsort (einer photometrischen Detektionseinrichtung) vorzusehen. Es versteht sich hierbei, dass dem Verbrennungsofen geeignete Mittel zur Bereitstellung und Zuführung eines Trägergases und einer Probe sowie eine Verbindung zwischen Ofenauslass und Gaskühler zugeordnet sind und dass die Anordnung schließlich auch eine photometrische Phosphor-Erfassungseinrichtung aufweist.

In einer zweckmäßigen Ausgestaltung dieser Vorrichtung ist vorgesehen, dass der Gaskühler zwei Kühlerstufen umfasst, wobei die Kühlfalle der ersten Kühlerstufe zugeordnet ist. Besonders einfach und leicht steuerbar und handhabbar ist die Anordnung in einer Ausführung, bei der der Gaskühler bzw. mindestens eine Kühlerstufe als elektrisch T-steuerbarer Peltier-Kühler ausgeführt ist.

Eine vorteilhafte Ausgestaltung der Kondensat-Transporteinrichtung zeichnet sich dadurch aus, dass sie eine koordinatengesteuerte und/oder durch einen Schrittmotor betriebene Injektionsspritze aufweist. Hierbei sind die Kühlfalle und die Kondensat-Transporteinrichtung zweckmäßigerweise so aufeinander abgestimmt, dass die Injektionsspritze von oben in ein (natürlich von oben zugängliches) Kondensat-Reservoir eintaucht. In gleicher Weise kann auch die Probenzuführung des Verbrennungsofens ausgestaltet sein, nämlich mit einer die Wasser- bzw. Abwasserprobe von oben einspritzenden Injektionsspritzeneinrichtung.

Im Übrigen ist der Verbrennungsofen in einer Ausführung der Erfindung als hermetisch verschließbarer Vertikalofen ausgeführt, wobei die Trägergaszuführung und Probezuführung im oberen Bereich und die Verbrennungsgas-/Trägergasableitung im unteren Bereich desselben angeordnet sind. Diese Verschließbarkeit wird durch das Vorsehen entsprechender Ventile in der Proben- und Trägergaszuführung sowie der Gasableitung sichergestellt.

In einer weiteren Ausführung der Erfindung umfasst die vorgeschlagene Anordnung neben den Mitteln zur Phosphorgehaltsbestimmung einen an einem Gasauslass des Gaskühlers angeordneten CO₂-Detektor zur Kohlenstoffgehaltsbestimmung und/oder einen NO-Detektor zur Stickstoffgehaltsbestimmung.

Vorteile und Zweckmäßigkeiten der Erfindung ergeben sich im Übrigen aus der nachfolgenden Beschreibung eines Ausführungsbeispiels und wesentlicher Ausführungsaspekte der Erfindung anhand der Figuren. Von diesen zeigen:
- Fig. 1: eine schematische Querschnittsdarstellung der wesentlichen Abschnitte eines Verbrennungsofens einer Anordnung gemäß einer Ausführungsform der Erfindung,
- Fig. 2: eine skizzenartige Gesamtdarstellung einer erfindungsgemäßen Anordnung und
- Fig. 3: eine Querschnittsdarstellung der zentralen Komponente des Gas-Flüssigkeits-Separators (Gaskühlers) der Anordnung nach Fig. 2.

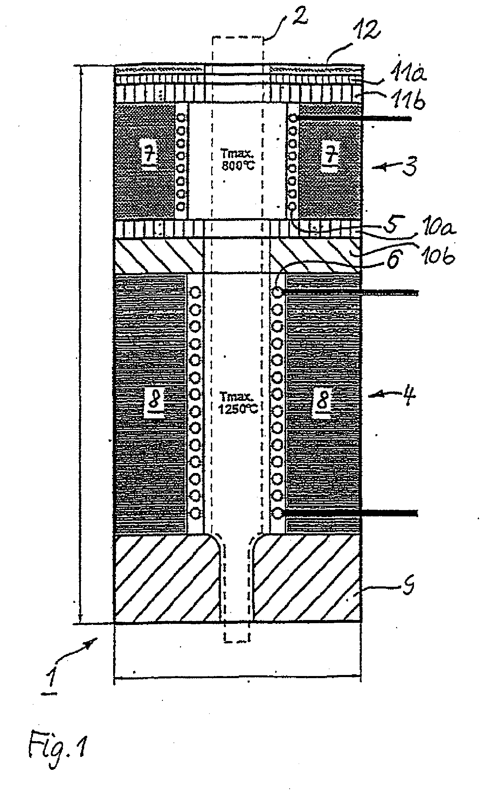

Fig. 1 zeigt in einer schematischen Querschnittsdarstellung wesentliche Abschnitte eines Probenverbrennungsofens 1 in einer zur Durchführung des erfindungsgemäßen Verfahrens geeigneten Ausführung, in den ein im wesentlichen langgestreckt zylindrisches Keramik-Reaktionsgefäß 2 (in der Figur mit einer gestrichelten Linie umrissartig gezeichnet) einsetzbar ist. Dieses hat am unteren Ende (kaltes Ofenende) einen rohrförmigen Auslaß mit einem Durchmesser im Bereich zwischen 6 und 10 mm, der zur Beseitigung von Salz-Ablagerungen leicht von unten her gereinigt werden kann.

Der Ofen 1 weist eine erste, obere Heizzone 3, in der bei dieser Ausführung eine Maximaltemperatur von 800°C erreicht werden kann, und eine zweite, untere Heizzone 4 auf, in der eine Maximaltemperatur von 1250°C erreicht wird. Beide Heizzonen werden über hohlzylindrisch um den entsprechenden Abschnitt des Reaktionsgefäßes 2 angeordnete Heizdrähte 5, 6 aus einer hochtemperaturfesten Speziallegierung, nämlich dem Material Kanthal-Fibrothal®, beheizt. Die obere ebenso wie die untere Heizzone weisen - wegen der unterschiedlichen Maximaltemperaturen unterschiedlich dicke - Keramikfaser-Isolationen 7 bzw. 8 auf, und auch der Fußbereich, der Bereich 10a, 10b zwischen den Heizzonen und der Bereich 11a, 11b unterhalb eines Aluminiumdeckels 12 sind keramikfaserisoliert. Eine (in der Figur nicht dargestellte) Probenbeschickungs- und Trägergaszuführungseinrichtung ist im Bereich oberhalb des Deckels 12 vorgesehen.

Der in Fig. 1 gezeigte und vorstehend beschriebene Ofenaufbau ermöglicht in vorteilhafter Weise die dauerhafte Realisierung speziell der in der zweiten, unteren Heizzone 4 erzeugten hohen Temperaturen, wobei die spezielle Isolierung sowohl zu einem vertretbaren Energieaufwand beiträgt als auch Gefährdungen der Umgebung ausschließt.

Eine diesem Verbrennungsofen zugeführte wässrige Probe wird durch eine katalysator-freie Verbrennung bei mindestens 1200°C, bevorzugt um 1250°C, in einer Weise aufgeschlossen, dass die darin enthaltenen verschiedenen Phosphor-Fraktionen sämtlich in Orthophosphat umgewandelt und somit einem Nachweis durch die bekannten und standardisierten Nachweisverfahren (insbesondere Blau- und Gelb-Methode) zugänglich werden, wie durch die Erfinder festgestellt werden konnte.

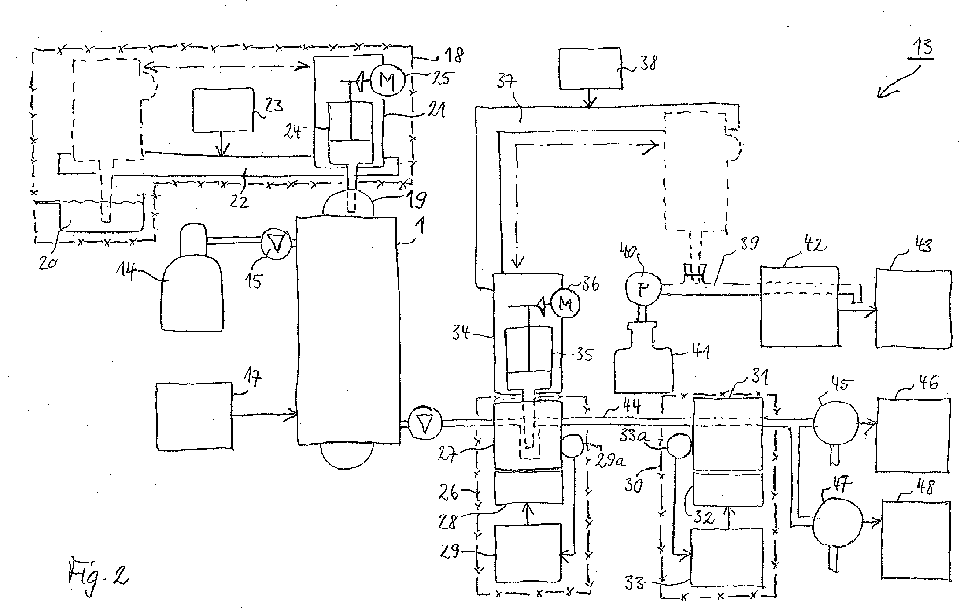

Fig. 2 zeigt in Art einer Prinzipskizze den Gesamtaufbau einer Messanordnung 13 zur Bestimmung verschiedener Wasserinhaltsstoffe von Abwasser bzw. Brauchwasser. Als Hauptkomponente dieser Anordnung 13 ist der in Fig. 1 dargestellte und oben beschriebene Verbrennungsofen 1 gezeigt; es kann aber auch ein anderer Typ von Verbrennungsofen (etwa mit Strahlungsheizung) an dessen Stelle eingesetzt sein. Im Interesse einer besseren Übersichtlichkeit der Darstellung sind nicht erfindungswesentliche Teile, die etwa der Kalibrierung und Reinigung der Messanordnung dienen, in dieser skizzenhaften Darstellung fortgelassen.

Ebenfalls nicht gezeigt ist eine Steuereinheit (Controller), die den Gesamtablauf des Probenaufschlusses und der Messvorgänge steuert und zu diesem Zwecke selbstverständlich mit den wesentlichen Absperr-, Transport-, Heiz- und Nachweiseinrichtungen der Anordnung verbunden ist. Die Implementierung und der Betrieb einer solchen Steuereinrichtung liegt, auf der Grundlage der hier gegebenen Verfahrensbeschreibung und des nachfolgend erläuternden Vorrichtungsaufbaus, im Rahmen fachmännischen Handelns.

Dem Verbrennungsofen 1 als einem Kernstück der Messanordnung 13 ist eingangsseitig ein Trägergasspeicher 14 zur Bereitstellung von Trägergas für die Messvorgänge, mit zugeordneter Eingangs-Ventileinrichtung 15 zugeordnet. Weiterhin hat der Ofen eine Heizsteuereinheit 17 zur Steuerung der elektrischen Ofenheizung und eine Probenzuführeinrichtung 18 zur Probenzuführung in ein Probeneinspritzventil 19 des Ofens.

Die Probenzuführeinrichtung 18 umfasst ein Probenreservoir 20, welches etwa am Einlauf einer Kläranlage angeordnet sein kann, eine Injektionseinheit 21, die auf einer Transportführung 22 verfahrbar gelagert ist und eine zugehörige Transportsteuerung 23. Die Spritzeneinheit 21 umfasst eine Dosierspritze 24 und einen Schrittmotor 25 zu deren präzise steuerbarer Betätigung und damit Dosierung eines vorbestimmten Probenvolumens.

Am Ausgang des Ofens 1 ist eine erste Kühlstufe 26 angeordnet, die eine Kühlfalleneinheit 27, einen Peltierkühler 28 und eine zugehörige Temperatursteuerung 29 mit T-Sensor 29a an oder in der Kühlfalleneinheit 27 umfasst. Stromabwärts der ersten Kühlstufe 26 ist eine zweite Kühlstufe 30 angeordnet, welche einen Kühlblock 31 mit zugeordnetem Peltierkühler 32 und eine diesen steuernde Temperatursteuereinheit 33 mit T-Sensor 33a umfasst.

Der ersten Kühlstufe 26 ist eine weitere Spritzeneinheit 34 zugeordnet, die - analog zur Spritzeneinheit 21 zur Beschickung des Verbrennungsofens 1 - eine Injektionsspritze 35 mit Schrittmotor 36 zu deren präzise gesteuerter Betätigung aufweist. Des Weiteren ist auch diese Spritzeneinheit 34 auf einer Transportführung 37 gelagert, der eine Transportsteuereinheit 38 zum Verfahren der Spritzeneinheit in eine zweite Betriebsposition zugeordnet ist. Diese befindet sich oberhalb einer Durchflussküvette 39, in die die Nadel der Injektionsspritze 35 ebenso eingreifen kann wie in die Kühlfalle 27. Diese zweite Betriebsstellung ist in der Figur, ebenso wie die Ausgangs-Betriebsstellung der Spritzeneinheit 21, gestrichelt dargestellt.

An einem Eingang der Durchflussküvette 39 ist über eine Pumpe 40 ein Reagensbehälter 41 angeschlossen, in dem eine für den photometrischen Phosphor-Nachweis benötigte Chemikalie bevorratet ist. Die Durchflussküvette 39 ragt in eine Photometereinheit 42, die zur photometrischen Analyse einer die Durchflussküvette 39 durchströmenden wässrigen Probe ausgebildet ist und deren Ausgang mit einer Phosphor-Auswertungsstufe 43 verbunden ist.

Am Ausgang der zweiten Kühlstufe 30 verzweigt sich die Ausgangsleitung 44 des Verbrennungsofens 1 zu einem NO-Detektor 45, der ausgangsseitig mit einer Stickstoff(TN)-Auswertungsstufe 46 verbunden ist, und zu einem CO₂-Detektor 47, welcher ausgangsseitig mit einer Kohlenstoff(TOC)-Auswertungsstufe 48 verbunden ist.

Die Funktionsweise der Messanordnung 13 ergibt sich weitgehend bereits aus den obigen Erläuterungen zum erfindungsgemäßen Verfahren, soll aber nachfolgend nochmals knapp zusammengefasst werden.

Mittels der ersten Spritzeneinheit 21 wird eine wässrige Probe aus dem Reservoir 20 entnommen, zum Verbrennungsofen 1 transportiert und in diesen eingespritzt. Sie wird bei den dort eingestellten Temperaturen augenblicklich verdampft und verbrannt, und das entstehende Verbrennungsgas wird mit einem aus dem Trägergas-Reservoir 14 gespeisten Trägergasstrom aus dem Ofen in die Ausgangsleitung 44 geführt. Im Kondensator wird der Verbrennungsgas-/Trägergasstrom auf eine erste Abkühltemperatur heruntergekühlt, bei der sich in der Kühlfalle 27 ein Kondensat abscheidet. Von diesem Kondensat wird mittels der zweiten Spritzeneinheit 34 eine vorbestimmte Menge abgezogen und in die Durchflussküvette 39 gebracht, wo es mit dem über die Pumpe 40 geförderten Reagens zum Bewirken eines photometrischen Nachweisvorganges vermischt und der Photometereinheit 42 zur Phosphor-Detektion zugeführt wird.

In der zweiten Kühlstufe 30 wir der Verbrennungsgas-/Trägergasstrom auf eine nahe 0°C liegende zweite Abkühltemperatur heruntergekühlt und ausgangsseitig der Kühlstufe den Gasdetektoren 44 und 46 für den NO- und CO₂-Nachweis zugeführt. Im Ergebnis der Nachweisvorgänge an den Detektoren 42, 45 und 47 ermittein die entsprechenden Auswertungsstufen 43, 46 und 48 den Gesamt-Phosphorgehalt (TP), den Gesamt-Stickstoffgehalt (TN) und den Gesamtgehalt an organischem Kohlenstoff (TOC) der wässrigen Probe, die aus dem Reservoir 20 entnommen und im Verbrennungsofen 1 aufgeschlossen wurde.

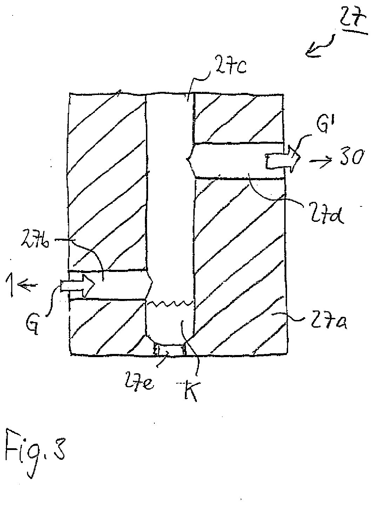

Fig. 3 zeigt den Aufbau der Kühlfalle 27 in einer Querschnittsdarstellung genauer. In einem Block 27a als Grundkörper ist ein Zuleitungsabschnitt 27b eingearbeitet, über den die Kühlfalle 27 mit dem Ausgang des Verbrennungsofens 1 in Verbindung steht und über den ein Verbrennungs-/Trägergasstrom G eintritt. Der Zuleitungsabschnitt 27b mündet in eine vertikale Bohrung 27c, in deren unterem Bereich sich beim Kühlen des Gasstromes ein Kondensat K absetzt. Im oberen Bereich des Grundkörpers 27a ist ein weiterer horizontaler Leitungsabschnitt 27d vorgesehen, der in die Bohrung 27c mündet und über den der abgekühlte und kondensatfreie Verbrennungsgas-Trägergasstrom G' schließlich zur zweiten Kühlstufe 30 geführt wird. Ein Stopfen 27e verschließt das untere Ende der Bohrung 27c.

Die Ausführung der Erfindung ist nicht auf das oben erläuterte Beispiel und die hier hervorgehobenen Aspekte beschränkt, sondern ebenso in einer Vielzahl von Abwandlungen möglich, die im Rahmen fachgemäßen Handelns liegen. So kann insbesondere die oben beschriebene zweistufige Kühlvorrichtung durch einen einfachen, einstufigen Gas-Flüssigkeits-Separator ersetzt sein, und auch hinsichtlich der Probenzuführeinrichtung mit der ersten Spritzeneinheit und/oder der Halterung und des Transportes der zweiten Spritzeneinheit sind Vereinfachungen im Interesse einer Kostensenkung, insbesondere durch Fortfall des entsprechenden elektronisch gesteuerten Transportmechanismus, möglich.

## Patentansprüche

1. Verfahren zur Bestimmung des Phosphorgehalts einer wässrigen Probe, insbesondere Abwasserprobe, wobei die Probe einem thermisch-oxidativen Aufschluss unterzogen und der Orthophosphat-Gehalt der aufgeschlossenen Probe in wässriger Analyse-Lösung photometrisch bestimmt wird,
**dadurch gekennzeichnet, dass**
der thermische Aufschluss einstufig durch katalysator-freie Verbrennung der Probe als Batch-Aufschluss in einem Verbrennungsofen ausgeführt, das entstehende Verbrennungsgas in einem Trägergasstrom aus dem Verbrennungsofen abgeführt und der Verbrennungs-/Trägergasstrom zur Gewinnung der wässrigen Analyse-Lösung als Kondensat hieraus gekühlt wird.

2. Verfahren nach Anspruch 1,
**dadurch gekennzeichnet, dass**
der gekühlte Verbrennungs-/Trägergasstrom zur Bestimmung des Kohlenstoff- und/oder Stickstoffgehaltes der Probe einem hierfür ausgebildeten CO₂- bzw. NO-Detektor zugeleitet wird.

3. Verfahren nach Anspruch 2,
**dadurch gekennzeichnet, dass**
die Phosphorgehalts-Bestimmung anhand der wässrigen Analyse-Probe und die Kohlenstoff- und/oder Stickstoffgehaltsbestimmung am Verbrennungs-/Trägergasstrom getrennt an verschiedenen aufgeschlossenen Proben, insbesondere alternierend an einer Folge von Proben, ausgeführt werden.

4. Verfahren nach Anspruch 2,
**dadurch gekennzeichnet, dass**
die Phosphorgehalts-Bestimmung anhand der wässrigen Analyse-Probe und die Kohlenstoff- und/oder Stickstoffgehaltsbestimmung am Verbrennungs-/Trägergasstrom an ein und denselben aufgeschlossenen Proben ausgeführt werden.

5. Verfahren nach einem der vorangehenden Ansprüche,
**dadurch gekennzeichnet, dass**
das Kondensat in einer Kühlfalle gesammelt und aus dieser eine definierte Menge für die photometrische Bestimmung abgezogen wird, wobei insbesondere das Abziehen von Kondensat aus der Kühlfalle und der Transport an einen Detektionsort mittels einer, insbesondere koordinatengesteuerten und/oder motorisch betätigten, Injektionsspritze ausgeführt wird.

6. Verfahren nach einem der vorangehenden Ansprüche,
**dadurch gekennzeichnet, dass**
die wässrige Analyse-Probe zur photometrischen Bestimmung einer Durchflussküvette oder einer anderen Detektionseinheit zugeführt wird.

7. Verfahren nach einem der vorangehenden Ansprüche,
**dadurch gekennzeichnet, dass**
der Verbrennungsgas-/Trägergasstrom mittels eines Peltier-Kühlers auf eine vorbestimmte Temperatur gekühlt wird.

8. Verfahren nach einem der vorangehenden Ansprüche,
**dadurch gekennzeichnet, dass**
der Verbrennungsgas-/Trägergasstrom zweistufig abgekühlt wird, wobei in einer ersten Stufe das Kondensat gewonnen wird und in einer zweiten Stufe eine Abkühlung bis nahe 0°C erfolgt.

9. Anordnung zur Bestimmung des Phosphorgehalts einer wässrigen Probe, insbesondere Abwasserprobe, wobei die Probe einem thermisch-oxidativen Aufschluss unterzogen und der Orthophosphat-Gehalt der aufgeschlossenen Probe in wässriger Analyse-Lösung photometrisch bestimmt wird, mit
- einem als Verbrennungsofen mit einer Trägergaszuführung, einer Probenzuführung und einer Verbrennungsgas-Trägergasableitung ausgeführten thermischen Reaktor,
- einer stromaufwärts an den Verbrennungsofen angeschlossenen Trägergasquelle,
- einem stromabwärts an den Verbrennungsofen angeschlossenen Gas-Flüssig-Separator zur Abscheidung eines Kondensats aus dem Verbrennungsgas-/Trägergasstrom,
- einer Kondensat-Transporteinrichtung zum Abziehen von Kondensat aus einer Kühlfalle und zu dessen Transport an einen Detektionsort und
- einer photometrischen Phosphor-Erfassungseinrichtung.

10. Anordnung nach Anspruch 9,
**dadurch gekennzeichnet, dass**
der Gaskühler zwei Kühlerstufen umfasst, wobei die Kühlfalle der ersten Kühlerstufe zugeordnet ist.

11. Anordnung nach Anspruch 9 oder 10,
**dadurch gekennzeichnet, dass**
der Gaskühler bzw. mindestens eine Kühlerstufe als elektrisch T-steuerbarer Peltier-Kühler ausgeführt ist.

12. Anordnung nach einem der Ansprüche 9 bis 11,
**dadurch gekennzeichnet, dass**
die Probenzuführung des Verbrennungsofens und/oder die Kondensat-Transporteinrichtung eine koordinatengesteuerte und/oder durch einen Schrittmotor betriebene Injektionsspritze aufweist, und dass insbesondere die Kühlfalle ein von oben zugängliches Kondensat-Reservoir aufweist und die Kondensat-Transporteinrichtung derart ausgeführt ist, dass die Injektionsspritze von oben in das Kondensat-Reservoir eintaucht.

13. Anordnung nach einem der Ansprüche 9 bis 12,
**dadurch gekennzeichnet, dass**
der Verbrennungsofen als hermetisch verschließbarer Vertikalofen ausgeführt ist, wobei die Trägergaszuführung und Probezuführung im oberen Bereich und eine Verbrennungsgas-/Trägergasableitung im unteren Bereich desselben angeordnet sind.

14. Anordnung nach einem der Ansprüche 9 bis 13,
**dadurch gekennzeichnet, dass**
die photometrische Phosphor-Erfassungseinrichtung eine Messküvette aufweist, die insbesondere zum Befüllen mit einer Injektionsspritze ausgebildet ist.

15. Anordnung nach einem der Ansprüche 9 bis 14,
**gekennzeichnet durch**
einen an einem Gasauslass des Gaskühlers angeordneten CO₂-Detektor zur Kohlenstoffgehaltsbestimmung und/oder einen NO-Detektor zur Stickstoffgehaltsbestimmung.

## Claims

1. Method for determining the phosphorus content of an aqueous sample, in particular drainage-water sample, wherein the sample is subjected to thermal-oxidative decomposition and the orthophosphate content of the decomposed sample in aqueous analysis solution is determined photometrically,
**characterized in that**
the thermal decomposition is carried out in a single step by catalyzer-free burning of the sample as batch-decomposition in a combustion oven, the resulting combustion gas is removed from the combustion oven in a carrier-gas stream, and the stream of combustion/carrier gas is cooled so as to obtain the aqueous analysis solution as a condensate therefrom.

2. Method according to claim 1,
**characterized in that**
to determine the carbon and/or nitrogen content of the sample the cooled combustion/carrier gas stream is conducted to a CO₂ and/or NO detector designed for this purpose.

3. Method according to claim 2,
**characterized in that**
the determination of phosphorus content with reference to the aqueous analysis sample, and that of the carbon and/or nitrogen contents from the combustion/carrier gas stream, are carried out separately with differently decomposed samples, in particular with a series of samples in alternation.

4. Method according to claim 2,
**characterized in that**
the determination of phosphorus content with reference to the aqueous analysis sample, and that of the carbon and/or nitrogen contents from the combustion/carrier gas stream, are carried out with one and the same decomposed samples.

5. Method according to one of the preceding claims,
**characterized in that**
the condensate is collected in a cooling trap and a specified amount is withdrawn therefrom for photometric detection,
wherein in particular the withdrawal of condensate from the cooling trap and its transport to a detection site are carried out by means of an injection syringe, in particular one that is coordinate-controlled and/or motor-actuated.

6. Method according to one of the preceding claims,
**characterized in that**
the aqueous analysis sample is transferred to a flow-through cuvette or to another detection unit for photometric detection.

7. Method according to one of the preceding claims,
**characterized in that**
the combustion/carrier gas stream is cooled by means of a Peltier cooler to a predetermined temperature.

8. Method according to one of the preceding claims,
**characterized in that**
the combustion/carrier gas stream is cooled in two stages, such that in a first stage the condensate is obtained and in a second stage cooling to nearly 0°C occurs.

9. Apparatus for determining the phosphorus content of an aqueous sample, in particular drainage-water sample, wherein the sample is subjected to thermal-oxidative decomposition and the orthophosphate content of the decomposed sample in aqueous analysis solution is determined photometrically, with
- a thermal reactor designed as a combustion oven with a carrier-gas inlet, a sample inlet and a combustion/carrier gas outlet,
- a carrier-gas source connected upstream to the combustion oven,
- a gas-liquid separator connected downstream to the combustion oven, to separate a condensate from the combustion/carrier gas stream,
- a condensate-transport device to withdraw condensate from a cooling trap and to transport it to a detection site, and
- a photometric phosphorus-detection device.

10. Apparatus according to claim 9,
**characterized in that**
the gas cooler comprises two cooler stages, such that the cooling trap is associated with the first cooler stage.

11. Apparatus according to claim 9 or 10,
**characterized in that**
the gas cooler or at least one cooler stage is constructed as an electrically T-controllable Peltier cooler.

12. Apparatus according to one of the claims 9 to 11,
**characterized in that**
the sample inlet of the combustion oven and/or the condensate-transport device comprises an injection syringe that is coordinate-controlled and/or driven by a stepping motor,
and that, in particular, the cooling trap comprises a condensate reservoir that is accessible from above, and the condensate-transport device is designed so that the injection syringe is inserted from above into the condensate reservoir.

13. Apparatus according to one of the claims 9 to 12,
**characterized in that**
the combustion oven is designed as a hermetically sealable vertical oven, such that the carrier-gas inlet and sample inlet are in the upper region, and an outlet for combustion/carrier gas is disposed in the lower region of the oven.

14. Apparatus according to one of the claims 9 to 13,
**characterized in that**
the photometric phosphorus-detection device comprises a measurement cuvette, which in particular is designed to be filled by means of an injection syringe.

15. Apparatus according to one of the claims 11 to 18,
**characterized by**
a CO₂ detector disposed at a gas outlet of the gas cooler to determine the carbon content and/or an NO detector to determine the nitrogen content.

## Revendications

1. Procédé destiné à déterminer la teneur en phosphore d'un échantillon aqueux, en particulier d'un échantillon d'eaux usées, sachant que l'échantillon est soumis à une décomposition thermique oxydante et la teneur en orthophosphate de l'échantillon décomposé est déterminée de manière photométrique dans une solution d'analyse aqueuse,
**caractérisé en ce que**
la décomposition thermique est effectuée en une étape par combustion exempte de catalyseur de l'échantillon sous forme de décomposition par lots dans un four de combustion, le gaz de combustion produit est évacué du four de combustion dans un courant de gaz porteur et le courant de gaz de combustion/gaz porteur est refroidi pour obtenir la solution d'analyse aqueuse sous forme de condensat.

2. Procédé selon la revendication 1,
**caractérisé en ce que**
pour déterminer la teneur en carbone et/ou en azote de l'échantillon, le courant de gaz de combustion/gaz porteur refroidi est amené à un détecteur de CO₂ ou de NO constitué à cet effet.

3. Procédé selon la revendication 2,
**caractérisé en ce que**
la détermination de la teneur en phosphore est effectuée sur la base de l'échantillon d'analyse aqueux et la détermination de la teneur en carbone et/ou en azote est effectuée au niveau du courant de gaz de combustion/gaz porteur de façon séparée sur divers échantillons décomposés, en particulier en alternance sur une séquence d'échantillons.

4. Procédé selon la revendication 2,
**caractérisé en ce que**
la détermination de la teneur en phosphore est effectuée sur la base de l'échantillon d'analyse aqueux et la détermination de la teneur en carbone et/ou en azote est effectuée au niveau du courant de gaz de combustion/gaz porteur sur les mêmes échantillons décomposés.

5. Procédé selon l'une des revendications précédentes,
**caractérisé en ce que**
le condensat est récolté dans un piège cryogénique et une quantité définie en est extraite pour la détermination photométrique, sachant qu'en particulier l'extraction de condensat à partir du piège cryogénique et l'acheminement à un lieu de détection sont effectués à l'aide d'une seringue d'injection, en particulier à commande à coordonnées et/ou à actionnement par moteur.

6. Procédé selon l'une des revendications précédentes,
**caractérisé en ce que**
pour la détermination photométrique, l'échantillon d'analyse aqueux est amené à une cuvette de circulation ou à une autre unité de détection.

7. Procédé selon l'une des revendications précédentes,
**caractérisé en ce que**
le courant de gaz de combustion/gaz porteur est refroidi à une température prédéterminée au moyen d'un refroidisseur Peltier.

8. Procédé selon l'une des revendications précédentes,
**caractérisé en ce que**
le courant de gaz de combustion/gaz porteur est refroidi en deux étapes, sachant que le condensat est obtenu à une première étape et un refroidissement jusqu'à près de 0°C a lieu à une deuxième étape.

9. Agencement destiné à déterminer la teneur en phosphore d'un échantillon aqueux, en particulier d'un échantillon d'eaux usées, sachant que l'échantillon est soumis à une décomposition thermique oxydante et la teneur en orthophosphate de l'échantillon décomposé est déterminée de manière photométrique dans une solution d'analyse aqueuse, comprenant
- un réacteur thermique exécuté comme four de combustion pourvu d'une arrivée de gaz porteur, d'une arrivée d'échantillon et d'une évacuation de gaz de combustion/gaz porteur,
- une source de gaz porteur raccordée en amont au four de combustion,
- un séparateur gaz-liquide raccordé en aval au four de combustion pour séparer un condensat du courant de gaz de combustion/gaz porteur,
- un dispositif d'acheminement de condensat pour l'extraction du condensat à partir d'un piège cryogénique et pour son acheminement à un lieu de détection et
- un dispositif de saisie de phosphore photométrique.

10. Agencement selon la revendication 9,
**caractérisé en ce que**
le refroidisseur de gaz comprend deux étages de refroidisseur, sachant que le piège cryogénique est associé au premier étage de refroidisseur.

11. Agencement selon la revendication 9 ou 10,
**caractérisé en ce que**
le refroidisseur de gaz ou au moins un étage de refroidisseur est exécuté comme refroidisseur Peltier à commande T électrique.

12. Agencement selon l'une des revendications 9 à 11,
**caractérisé en ce que**
l'arrivée d'échantillon du four de combustion et/ou le dispositif d'acheminement de condensat présentent une seringue d'injection à commande à coordonnées et/ou à actionnement par moteur pas-à-pas, et **en ce qu'**en particulier le piège cryogénique présente un réservoir de condensat accessible par le haut et le dispositif d'acheminement de condensat est exécuté de telle façon que la seringue d'injection s'immerge par le haut dans le réservoir de condensat.

13. Agencement selon l'une des revendications 9 à 12,
**caractérisé en ce que**
le four de combustion est exécuté comme four vertical à obturation hermétique, sachant que l'arrivée de gaz porteur et l'arrivée d'échantillon sont disposées dans la zone supérieure et une évacuation de gaz de combustion/gaz porteur est disposée dans la zone inférieure de celui-ci.

14. Agencement selon l'une des revendications 9 à 13,
**caractérisé en ce que**
le dispositif de saisie de phosphore photométrique présente une cuvette de mesure qui est constituée en particulier pour être remplie au moyen d'une seringue d'injection.

15. Agencement selon l'une des revendications 9 à 14,
**caractérisé par**
un détecteur de CO₂ disposé au niveau d'une sortie de gaz du refroidisseur de gaz pour la détermination de la teneur en carbone et/ou un détecteur de NO pour la détermination de la teneur en azote.
